# EUROPEAN PATENT SPECIFICATION

(11) **EP 2 215 964 B1**
(45) Date of publication and mention of the grant of the patent: **21.12.2016**
(21) Application number: 10250189.7
(22) Date of filing: 04.02.2010
(51) Int. Cl.: A61B 5/1473, G01N 27/30

(54) **Flexible indwelling biosensor, flexible indwelling biosensor insertion device**
Flexibler Dauerbiosensor, Vorrichtung zum Einsetzen des flexiblen Dauerbiosensors
Biocapteur flexible à demeure, dispositif d'insertion du biocapteur flexible à demeure

(30) Priority: 05.02.2009 US 366466
(43) Date of publication of application: 11.08.2010
(73) Proprietor: Lifescan, Inc., Wayne, PA 19087 (US)
(72) Inventor: Krulevitch, Peter, Pleasanton, CA 94566 (US); Savage, Donna, Rolling Hills Estates, CA 90274 (US); Olson, Lorin P., Scotts Valley, CA 95066 (US); Bowman, Leif, Livermore, CA 94550 (US)
(74) Representative: Tunstall, Christopher Stephen

(56) References cited:
- EP-A1- 2 055 333
- WO-A1-99/33504
- WO-A1-2007/091633
- WO-A2-2007/028138
- WO-A2-2008/051920
- JP-A- 2004 208 777
- US-A1- 2002 032 374

## Description

### BACKGROUND OF THE INVENTION

### Field of the Invention

The present invention relates, in general, to medical devices and, in particular, to indwelling biosensors, related insertion devices, and related methods.

### Description of Related Art

A variety of indwelling biosensors are of interest to the scientific and medical community. For example, indwelling biosensors for continuous glucose monitoring have recently become available. These biosensors are subcutaneously inserted below a user's skin using a separate insertion device (e.g., a rigid hollow needle). The separate insertion device is removed before the biosensor is employed to continuously measure glucose concentrations in the user's interstitial fluid for an extended period of time (for example, seven days).

### BRIEF DESCRIPTION OF THE DRAWINGS

The novel features of the invention are set forth with particularity in the appended claims. A flexible indwelling biosensor with all the features ofthe preamble to claim 1 is known from WO2007/0916331. WO2007/0268138 discloses insertion devices.

A better understanding ofthe features and advantages ofthe present invention will be obtained by reference to the following detailed description that sets forth illustrative embodiments, in which the principles of the invention are utilized, and the accompanying drawings, in which like numerals indicate like elements, of which:
FIG. 1A is a simplified depiction of a portion of a flexible indwelling biosensor;
FIG. 1B is a simplified cross-sectional depiction of the portion of a flexible indwelling biosensor of FIG. 1A taken along line B-B of FIG. 1A;
FIG. 1C is a simplified cross-sectional depiction of the portion of a flexible indwelling biosensor of FIG. 1A taken along the length of FIG. 1A;
FIG. 1D is a simplified cross-sectional depiction illustrating an alternative placement of a sensing element in a flexible indwelling biosensor otherwise as depicted in FIG. 1A;
FIG. 1E is a simplified cross-sectional depiction illustrating of the addition of a framework opening through the body portion of a flexible indwelling biosensor otherwise as depicted in FIG. 1D;
FIG. 2A is a simplified cross-sectional depiction of a portion of a flexible indwelling biosensor according to an embodiment of the present invention;
FIG. 2B is a simplified cross-sectional depiction of the portion of a flexible indwelling biosensor of FIG. 2A taken along line B-B of FIG. 2A;
FIG. 2C is a simplified cross-sectional depiction of the portion of a flexible indwelling biosensor of FIG. 2A taken along line C-C of FIG. 2A;
FIG. 3 is a simplified perspective view of the distal end of a flexible indwelling biosensor (with dashed lines indicating a channel and signal transmitting line of the biosensor that are hidden from view in the perspective of FIG. 6) according to another embodiment;
FIG. 4 is a simplified perspective view of a flexible indwelling biosensor according to another embodiment;
FIG. 5 is a simplified perspective depiction of a flexible indwelling biosensor insertion device according to an embodiment of the present invention prior to insertion of the flexible indwelling biosensor into a target site (not shown) and in the absence of a wireless transmitter;
FIG. 6 is a simplified perspective depiction of the flexible indwelling biosensor insertion device of FIG. 5 after the flexible indwelling biosensor has been inserted into a target site (not shown);
FIG. 7 is a simplified perspective depiction of the flexible indwelling biosensor insertion device of FIG. 6 after a wireless transmitter has been removably attached thereto;
FIG. 8 is a simplified, exploded perspective depiction of the flexible indwelling biosensor of FIG. 5 and a portion of the wireless transmitter of FIG. 7;
FIG. 9 is a simplified cross-sectional depiction of a portion of the flexible indwelling biosensor insertion device of FIG. 7 including portions of the removably attached wireless transmitter; and
FIG. 10 is a flow diagram depicting stages in a process for inserting a flexible indwelling biosensor into a target site according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

Flexible indwelling biosensors according to embodiments of the present invention include an elongated framework formed from a flexible material (e.g., a Nitinol strip) with a body portion, a sharp head, a distal end and a proximal end. Such flexible indwelling biosensors also include a biosensor (such as a glucose sensor) integrated with the elongated framework with the biosensor having a sensing element disposed over at least one of the body portion or sharp head of the elongated framework (for example, disposed on and/or suspended over the body portion or elongated framework). Moreover, the sharp head is disposed at the distal end of the elongated framework and the sharp head and at least the sensing element of the biosensor are configured for insertion into a target site (for example, a subcutaneous target site). Further features, characteristics and benefits of such flexible indwelling biosensors are described below with respect to various drawings.

One skilled in the art will recognize that a biosensor is a device that detects and produces a signal related to a physiological change, process or analyte (such as information regarding glucose concentration in interstitial fluid). Such biosensors include those based on enzymatic reactions combined with electrochemical or spectroscopic transduction techniques. Relevant, but non-limiting, examples of biosensors are described in U.S. Patents 7,471,972 B2, 7,344,500 B2, and 6,990,366 B2, each of which is hereby incorporated by reference as if fully set forth.

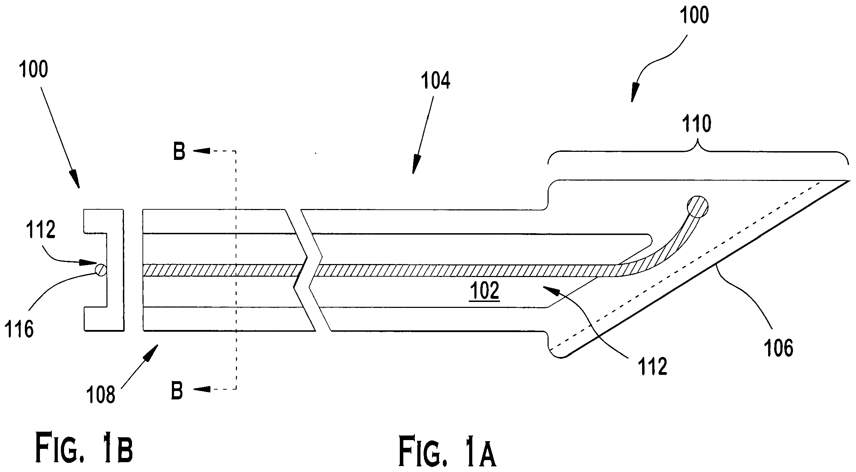

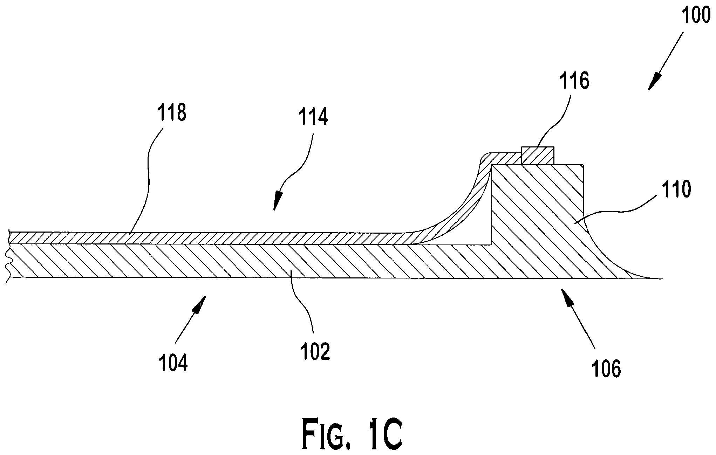

FIGs. 1A, 1B and 1C depict, in a simplified manner, portions of a flexible indwelling biosensor 100. Referring to FIGs. 1 A through 1C, flexible indwelling biosensor 100 includes an elongated strip 102 (i.e., an elongated framework) formed from a flexible material (such as a Nitinol material, other suitable flexible or superelastic material) with a body portion 104, a distal end 106, a proximal end 108, a sharp head 110 disposed at distal end 106 and a channel 112. Channel 112 extends along the length of the elongated strip. Sharp head 110 is configured for subcutaneous skin insertion. FIG. 1A illustrates an embodiment in which only a single edge of sharp head 110 is sharp (as indicated by the dashed line of FIG. 1A).

Flexible indwelling biosensor 100 also includes a biosensor 114 (shown in cross-hatching) that has a sensing element 116 and a signal transmitting line 118. Once apprised of the present invention, one skilled in the art will recognize that biosensor 114 can include a signal transmitting line of any suitable type including, for example, a co-axial cable, optical cable, a paired two-wire line, or a three-wire line. Moreover, biosensors employed in flexible indwelling biosensors according to embodiments of the present invention can transmit signals using wireless methodologies including those that employ radio frequency (RF) and capacitive coupling techniques. In the embodiment of FIGs. 1A-1C, signal transmitting line 118 runs the length of the elongated strip 102 in channel 112.

Biosensor 114 is integrated with the elongated framework and sensing element 116 is securely positioned on sharp head 110 using, for example, a suitable adhesive (not shown). Moreover, both sharp head 110 and sensing element 116 are configured for insertion into a subcutaneous target site.

Flexible indwelling biosensors according to embodiments of the present invention are beneficial in that, for example, they can be consistently inserted to a predetermined depth below the skin, are comfortably flexible while being kink-resistant, and have a relatively small cross-sectional area.

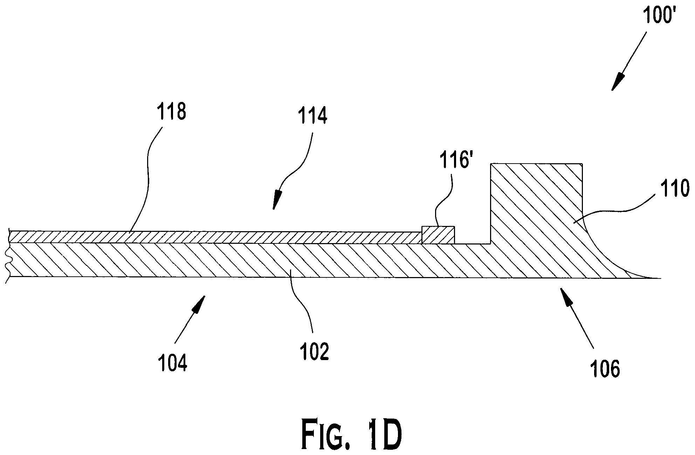

FIG. 1D is a simplified cross-sectional depiction illustrating an alternative placement of a sensing element 116' in a flexible indwelling biosensor 100' that is otherwise essentially similar to flexible indwelling biosensor 100 depicted in FIG. 1A. In the embodiment of FIG. 1D, sensing element 116' is disposed within channel 112 near sharp head 110. The configuration of FIG. 1D reduces the frontal profile of the flexible indwelling biosensor and, therefore, beneficially reduces the likelihood that the sensing element will impede insertion of the sharp head into a target site and also reduces the likelihood that the sensing element will become dislodged during insertion.

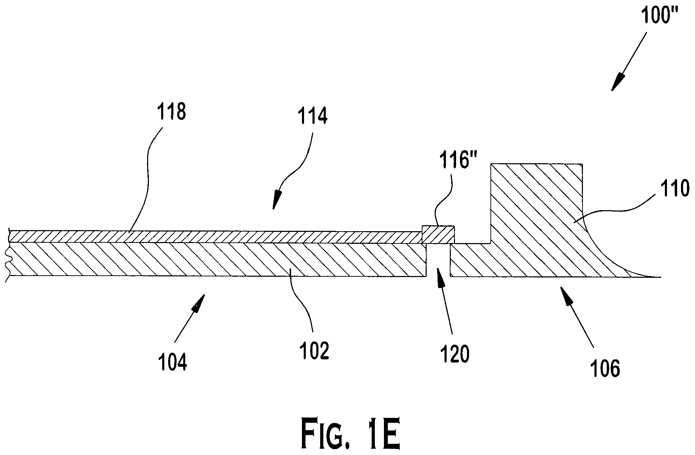

FIG. 1E is a simplified cross-sectional depiction of a flexible indwelling biosensor 100" that has been modified by the addition of a framework opening 120 through the body portion 104 of elongated strip 102 below sensing element 116' but is otherwise essentially similar to flexible indwelling biosensor 100' of FIG. 1D. Framework opening 120 is beneficially configured to increase the exposure of sensing element 116' to interstitial fluid, blood or other bodily fluid when the flexible indwelling biosensor 100"is inserted into a target site.

Once apprised of the present disclosure, one skilled in the art will recognize that sensing elements employed in flexible indwelling biosensors according to embodiments of the present invention can be generally disposed on and/or suspended over the body portion or the sharp head of the elongated framework. FIGs. 1A-1E depict the sensing element on a sharp head (FIGs. 1A-1C) or on the body portion (FIGs. 1D and 1 E). However, other configurations are possible including, for example, a configuration wherein the body portion of the elongated framework is cylindrical in shape with the transmission line of the biosensor being wound around the cylindrical elongated framework.

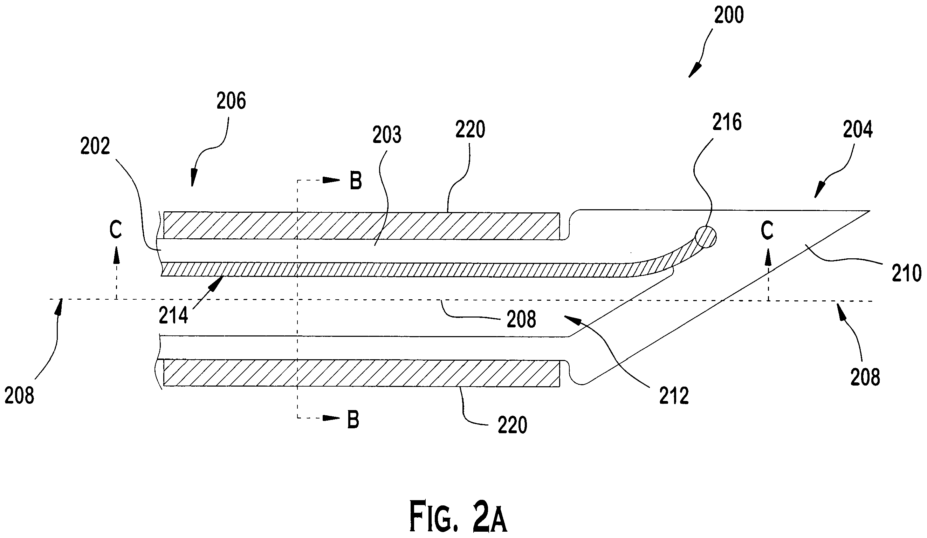

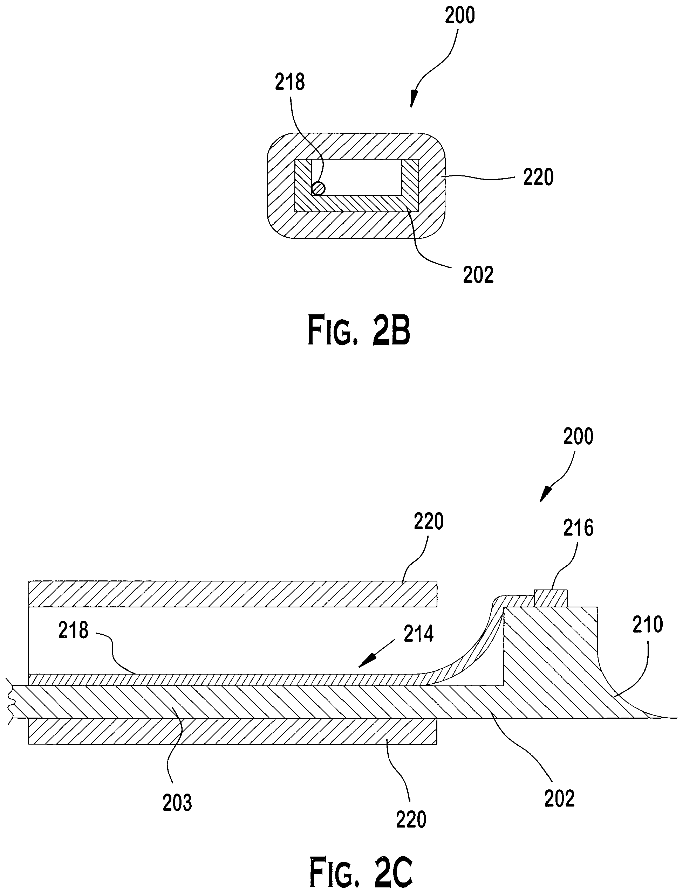

FIGs. 2A, 2B and 2C depict, in a simplified manner, a portion of a flexible indwelling biosensor 200 according to the present invention. Referring to FIGs. 2A through 2C, flexible indwelling biosensor 200 includes an elongated strip 202 formed from a flexible material (such as a Nitinol, other suitable flexible or superelastic material) with a body portion 203, a distal end 204, a proximal end 206, a longitudinal axis 208 (depicted by a dashed line), a sharp head 210 disposed at distal end 204 and a channel 212. Channel 212 is disposed parallel to (for example, along) the longitudinal axis 208. Sharp head 210 is configured for subcutaneous skin insertion.

Flexible indwelling biosensor 200 also includes a biosensor 214 that has a sensing element 216 and a signal transmitting line 218. In addition, flexible indwelling biosensor 200 includes a flexible tube 220 at least partially jacketing elongated strip 202 and biosensor 214 between distal end 204 and proximal end 206. Flexible tube 220 serves, at least, to secure and contain signal transmitting line 218. Moreover, flexible tube 220 can, if desired, be configured to provide a liquid-tight seal between the flexible tube, the biosensor and the elongated framework within the channel, thus preventing inadvertent flow of liquid through channel 212. The shape of flexible tubes
employed in embodiments of the present invention is configured such that they do
not completely encircle the elongated framework, whereby flexible tube 220 has a C-shaped cross section with a longitudinal opening (i.e., the open portion of the "C" cross-section) that is aligned with channel 212 or otherwise provides for a sensing element to be exposed.

If desired, flexible indwelling biosensor 200 can be partially coated with a lubricious material to facilitate insertion into a user's target site (for example, subcutaneous skin insertion). In addition, the elongated framework can be configured as a component of the biosensor (e.g., as one wire of a two- or three-wire signal transmitting line), thus simplifying the flexible indwelling biosensor design. Such configuration can include, for example, suitably coating the elongated framework with an electrically nonconducting layer.

Since flexible indwelling biosensors according to embodiments of the present invention can be formed with an elongated framework that is flexible and kink-resistant, they can have a relatively small cross-sectional area. It is hypothesized, without being bound, that such small cross-sectional areas result in minimal subcutaneous insertion pain and will be comfortable to wear.

Nitinol employed in embodiments of the present invention can be beneficially pre-processed (also referred to as preprogrammed) using techniques known to one skilled in the art to possess a variety of superelastic characteristics that are also known to those of skill in the art (such as, for example, kink-resistance, the ability to accommodate large loads and the ability to return to an original (preprogrammed) shape following release of mechanically deforming stresses).

Flexible indwelling biosensors 100 and 200 are very flexible, especially when bending such that the open side of their channels face towards (or away from) the center of the radius of curvature, referred to as the flexible bending direction. Moreover, use of superelastic materials (such as Nitinol with a Young's modulus of in the range of approximately 35 to 75 GPa) provide for flexible indwelling biosensors 100 and 200 to bend considerably without kinking.

In the embodiment of FIGs. 1A-1C and 2A-2C, the elongated framework has a C-shaped cross-section (see FIG. 1B in particular). However, once apprised of the present disclosure, one skilled in the art will recognize that other suitable elongated strip cross-section shapes can be used to control (i.e., predetermine) the amount of flexibility in different directions. Moreover, the elongated framework employed in embodiments of the present invention can have a cross section shape that changes along the length of the flexible medical device conduit to provide for varying flexibility along the length.

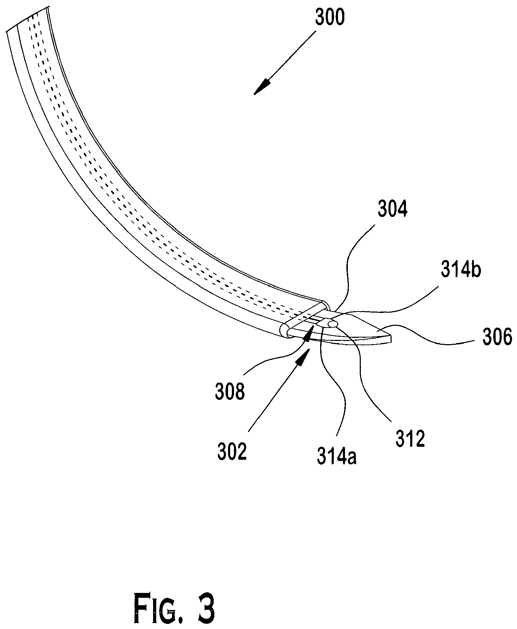

FIG. 3 is a simplified perspective view of the distal end of a flexible indwelling biosensor 300 according to another embodiment. Flexible indwelling biosensor 300 includes a curved elongated framework 302 formed from a flexible material (such as Nitinol). Curved elongated framework 302 includes a body portion 304 and a sharp head 306. Curved elongated framework 302 also includes a channel 308 disposed along the longitudinal axis of curved elongated framework 302.

Flexible indwelling biosensor 300 further includes a biosensor 310 that is integrated with curved elongated framework 302. Biosensor 310 includes a sensing element 312 disposed on sharp head 306 and two signal transmission lines 314a and 314b (depicted as a single dashed line within channel 308) that are partially contained within channel 308. Once apprised of the present disclosure, one skilled in the art will recognize that sensing elements employed in flexible indwelling biosensors according to the present invention can be also be disposed on the body portion of the elongated framework.

In the embodiment of FIG. 3, sharp head 306 is disposed at the distal end of curved elongated framework 302. Moreover, sharp head 306 and sensing element 312 are configured for insertion into a target site.

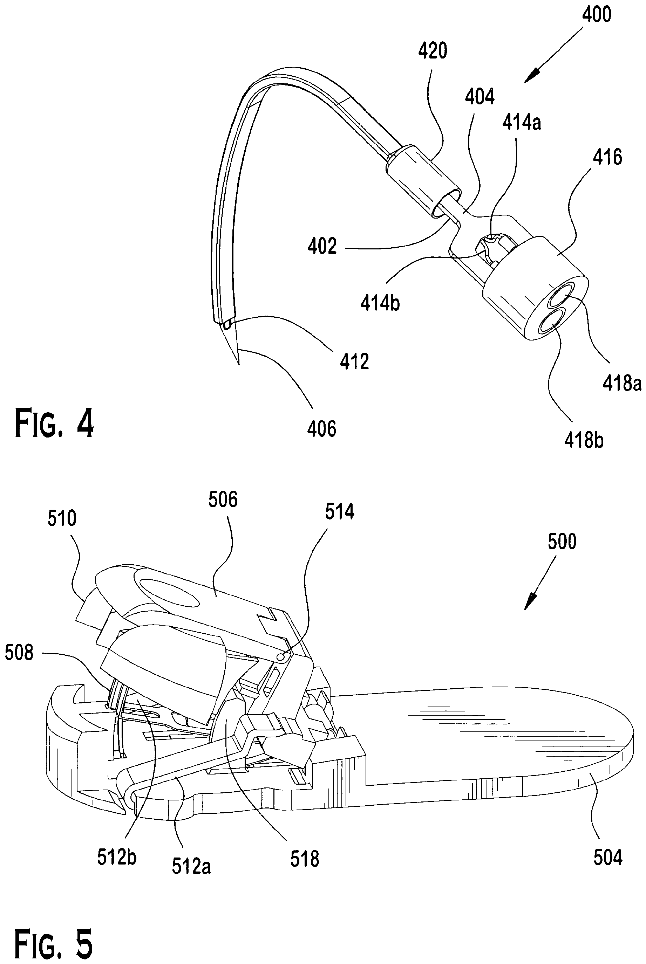

FIG. 4 is a simplified perspective view of a flexible indwelling biosensor 400 according to yet another embodiment. Flexible indwelling biosensor 400 includes a curved elongated framework 402 formed from a flexible material (such as Nitinol). Curved elongated framework 402 includes a body portion 404 and a sharp head 406 at the distal end of the curved elongated framework. Although FIG. 4 depicts an elongated framework that is curved in a freestanding state, once apprised of the present disclosure, one skilled in the art will recognize that elongated frameworks employed in embodiments of the present invention can be, if desired, straight in a freestanding state.

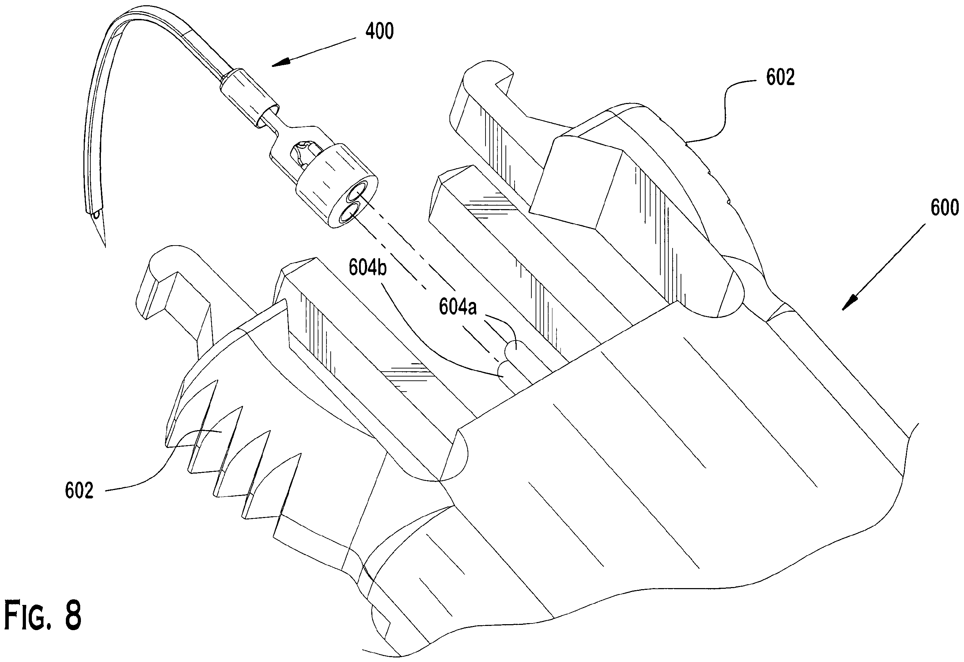

Flexible indwelling biosensor 400 further includes a biosensor that is integrated with curved elongated framework 402. The biosensor includes a sensing element 412 (such as an interstitial fluid glucose sensing element) disposed on sharp head 406, two signal transmission lines 414a and 414b, and a transmission line connector 416. Transmission line connector 416 includes electrical contacts 418a and 418b. As will be described further with respect to FIG. 8 below, electrical contacts 418a and 418b are configured for operational engagement with terminal pins of a wireless transmitter. Flexible indwelling biosensor 400 also includes a polymer jacket 420.

Methods for manufacturing flexible frameworks suitable for use in flexible indwelling biosensors according to embodiments of the present invention include etching a channel into an elongated Nitinol strip and forming a sharp head on a distal end of the elongated Nitinol strip. Alternatively, stamping and/or coining techniques can be employed to form the channel and sharp head of embodiments of the current invention. Moreover, conventional sharpening techniques, such as grinding, can also be used to form the sharp head.

A flexible indwelling biosensor according to embodiments of the present invention can be formed, for example, from an etched elongated Nitinol strip (with a sharp head) with a heat shrunk poly(tetrafluoroethylene) or PTFE polymer jacket serving as a flexible tube.

Flexible indwelling biosensor insertion devices according to the present invention include a flexible indwelling biosensor and an insertion mechanism. The flexible indwelling biosensor of such devices includes an elongated framework formed from a flexible material (e.g., a Nitinol strip) with a body portion, a sharp head, a distal end, and a proximal end. The flexible indwelling biosensor also includes a biosensor (such as a glucose biosensor) integrated with the elongated framework, the biosensor having a sensing element positioned on at least one of the body portion or the sharp head of the elongated framework. Moreover, the sharp head is disposed at the distal end of the elongated framework and the sharp head and at least the sensing element of the biosensor are configured for insertion into a target site (for example, a subcutaneous target site). In addition, the insertion mechanism is operatively connected to, and integrated with, the flexible indwelling biosensor, and configured to insert a portion of the flexible indwelling biosensor, including at least the sharp head and the sensing element, into the target site.

Flexible indwelling biosensor insertion devices according to embodiments of the present invention provide for the sharp head to be beneficially obscured from view and from unintentional contact with surfaces during insertion and for insertion to occur easily and with minimal steps. Further features, characteristics and benefits of such flexible indwelling biosensor insertion devices are described below with respect to various drawings.

Furthermore, flexible indwelling biosensors suitable for employment in flexible indwelling biosensor insertion devices according to embodiments of the present invention have been described above (for example, with respect to FIGs. 1A-1C, 2A-2C, 3 and 4). Exemplary embodiments of insertion mechanisms employed in flexible indwelling biosensor insertion devices according to embodiments of the present invention are described below. In this respect it should be noted that the flexible indwelling biosensor is integrated with the insertion mechanism in that the flexible indwelling biosensor is not, and cannot readily be, removed, separated or discarded from the insertion mechanism during patient use.

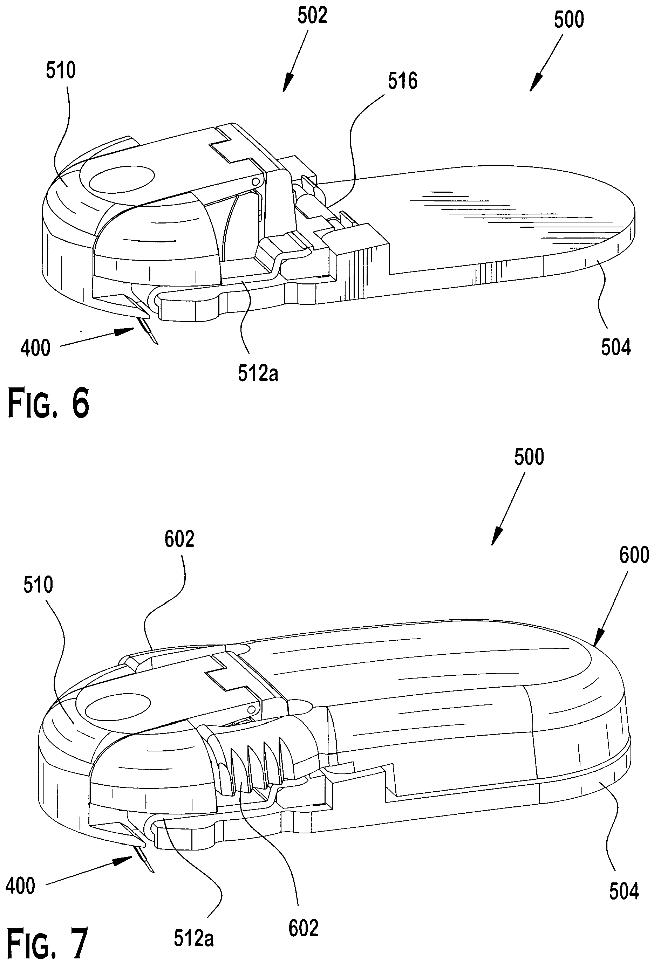

FIG. 5 is a simplified perspective depiction of a flexible indwelling biosensor insertion device 500 according to an embodiment of the present invention that includes the flexible indwelling biosensor of FIG. 4. FIG. 5 depicts flexible indwelling biosensor insertion device 500 prior to insertion of the flexible indwelling biosensor into a target site (not shown) and in the absence of a wireless transmitter. FIG. 6 is a simplified perspective depiction of flexible indwelling biosensor insertion device 500 after the flexible indwelling biosensor has been inserted into a target site (not shown) and FIG. 7 is a simplified perspective depiction of flexible indwelling biosensor insertion device 500 after a wireless transmitter has been removably attached thereto.

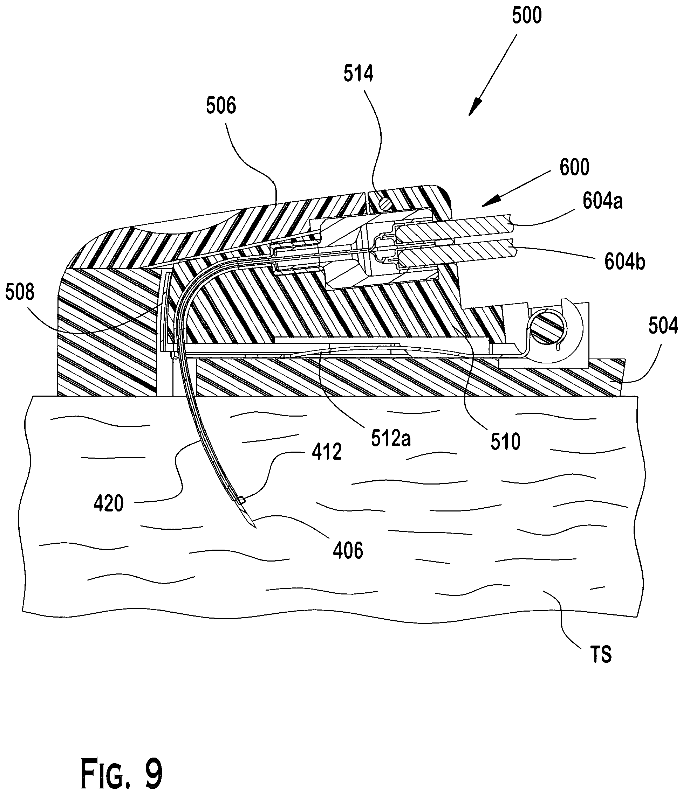

FIG. 8 is a simplified, exploded perspective depiction of the flexible indwelling biosensor employed in flexible indwelling biosensor insertion device 500 and a portion of the wireless transmitter of FIG. 7. FIG. 9 is a simplified cross-sectional depiction of a portion of the flexible indwelling biosensor insertion device of FIG. 7 including portions of the removably attached wireless transmitter.

Referring to FIGs. 4 through 9, flexible indwelling biosensor insertion device 500 includes a flexible indwelling biosensor 400 (previously described with respect to FIG. 4) and an insertion mechanism 502. Insertion mechanism 502 is operatively connected to, and integrated with, flexible indwelling biosensor 400. Moreover, as is described further below, insertion mechanism 502 is configured to insert a portion of the flexible indwelling biosensor, including at least the sharp head and the sensing element, into a target site TS (see, in particular, FIG. 9).

Insertion mechanism 502 includes a platform 504, button 506, guide 508, upper housing 510, springs 512a and 512b, button hinge 514, upper housing hinge 516 and at least one prop 518. Wireless transmitter 600 (depicted in FIGs. 7, 8, and 9) includes latches 602 and electrical terminals 604a and 604b.

Insertion mechanism 502 is configured such that flexible indwelling biosensor 400 is automatically inserted into a target site (TS) by a spring-loaded mechanism when a user presses button 506. Further details of operations are described immediately below.

In FIG. 5, platform 504 is adhered against a user's skin at a target site in an undeployed position. Upper housing 510 holds the proximal end of flexible indwelling biosensor 400 and upper housing 510 is held in the up position of FIG. 5 by prop 518. Springs 512a and 512b are preloaded and press against upper housing 510. Pressing button 506 causes button 506 to rotate about button hinge 514, releasing upper housing 510 from prop 518. Springs 512a and 512b then pull down upper housing 510 to the deployed position of FIG. 6.

Guide 508 prevents flexible indwelling biosensor 400 from buckling during insertion into a target site. When moving from the undeployed position of FIG. 5 to the deployed position of FIG. 6, upper housing 510 rotates about upper housing hinge 516. If desired, latches can be employed to secure upper housing 510 to platform 504 in the deployed position. In the deployed position, sharp head 406 of flexible indwelling biosensor 400 extends beyond the lower surface of platform 504, penetrating the user's skin.

FIGs. 7 and 9 depict flexible indwelling biosensor insertion device 500 after a wireless transmitter 600 has been attached and removabely secured with latches 602. Wireless transmitter 600 can be removed by pressing on latches 602.

The curved shape of flexible indwelling biosensor 400 and rotating action of upper housing 510 enable flexible indwelling biosensor 400 to pierce the skin with sharp head 406 perpendicular to the skin's surface, which may reduce the probability of bleeding. However, flexible indwelling biosensor 400 curves as it enters the skin, allowing for precise depth placement of the sensing element 412, and for a sufficient length of flexible indwelling biosensor 400 to reside below the surface of the skin to prevent it from inadvertently being pulling out. Alternatively, a straight elongated framework can be employed to provide for a straight perpendicular or straight angled insertion of the flexible indwelling biosensor into a target site (such as a user's skin target site).

The sharp head of flexible medical device conduits according to embodiments of the present invention remains in the target site during use of the flexible indwelling biosensor (for example, during the detection of glucose in interstitial fluid) and is only removed, for example, when the entire flexible indwelling biosensor is removed from the target site. Since the flexible indwelling biosensor is highly flexible (for example, being formed of Nitinol and, optionally, a flexible polymer tube), it can remain inserted without undue pain or discomfort during use.

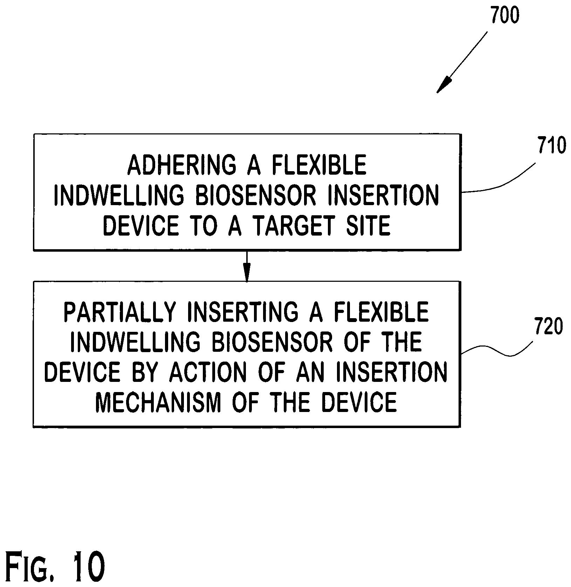

FIG. 10 is a flow diagram depicting stages in a method 700 for inserting a flexible indwelling biosensor into a target site according to an embodiment of the present invention. Method 700 includes, at step 710, adhering a flexible indwelling biosensor insertion device (that includes a flexible indwelling biosensor and an integrated insertion mechanism), to a target site (e.g., a user's skin target site). The flexible indwelling biosensor of the flexible indwelling biosensor insertion device has been described herein with respect to flexible indwelling biosensors according to the present invention including (for example, those of FIGs. 1A-1C, 2A-2C, 3 and 4) and the insertion mechanism has been described with respect to flexible indwelling biosensor insertion devices according to the present invention (for example, the device of FIGs. 5-9).

The flexible indwelling biosensor is then partially inserted into the target site by action of the insertion mechanism, as set forth in step 720. If desired, a wireless transmitter can be removably attached to the flexible indwelling biosensor following step 720.

Method 700 has fewer steps and is simpler than conventional indwelling biosensor insertion techniques. Therefore, it is expected that the method will have a higher rate of success than conventional methods. Also, flexible biosensor insertion methods according to the present invention do not involve the use of a sharp insertion tool that must be removed immediately after use for insertion of an indwelling biosensor.

Once apprised of the present disclosure, one skilled in the art will recognize that method 700 can be readily modified to incorporate any of the procedures, uses, methodologies and actions described herein with respect to flexible indwelling biosensors and flexible indwelling biosensor insertion devices according to embodiments of the present invention.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims and their equivalents be covered thereby.

## Claims

1. A flexible indwelling biosensor (200) comprising:
an elongated framework (202) formed from a flexible material, the elongated framework having:
a body portion (203);
a sharp head (210);
a distal end (204); and
a proximal end (206); and
a biosensor (214) integrated with the elongated framework, wherein
the elongated framework is an elongated strip with a longitudinal axis extending from the distal end to the proximal end; and
wherein the elongated strip has at least one channel (212) formed therein, the at least one channel disposed at least partially parallel to the longitudinal axis; and
wherein the biosensor is at least partially contained with in the at least one channel;
the biosensor including:
a sensing element (216) disposed over at least one of the body portion or sharp head of the elongated framework;
wherein the sharp head is disposed at the distal end of the elongated framework; and
wherein the sharp head and at least the sensing element of the biosensor are configured for insertion into a target site;
the flexible indwelling biosensor further including a flexible tube at least partially jacketing the elongated framework and the biosensor between the distal end and the proximal end of the elongated framework,
**characterised in that** the flexible tube has a C-shaped cross section such that the flexible tube partially encircles the elongated framework.

2. The flexible indwelling biosensor of claim 1 wherein the flexible tube is configured to provide a liquid-tight seal between the flexible tube, the biosensor and the elongated framework within the channel.

3. The flexible indwelling biosensor of claim 1 wherein the biosensor includes at least one signal transmission line, preferably two or three signal transmission lines, each having:
a proximal end; and
a signal transmission connector disposed at the proximal end of the signal transmission line.

4. The flexible indwelling biosensor of claim 1 wherein the sharp head is configured for subcutaneous insertion and the target site is a skin target site.

5. The flexible indwelling biosensor of claim 1 wherein the flexible material is a superelastic flexible material, for example Nitinol.

6. The flexible indwelling biosensor of claim 1 wherein the sensing element is disposed on or suspending over at least one of the sharp head and the body portion of the elongated framework.

7. The flexible indwelling biosensor of claim 1 or claim 2 wherein the sensing element is disposed within the channel and on or suspended over the body portion of the elongated framework.

8. The flexible indwelling biosensor of claim 1 wherein the body portion of the elongated framework includes a framework opening therethrough and the sensing element is disposed over the framework opening.

9. A flexible indwelling biosensor insertion device (500) comprising:
a flexible indwelling biosensor (200) according to any preceding claim; and
an insertion mechanism (502) operatively connected to, and integrated with, the flexible indwelling biosensor, the insertion mechanism configured to insert a portion of the flexible indwelling biosensor, including at least the sharp head and the sensing element, into a target site.

10. The flexible indwelling biosensor insertion device of claim 9 further including a wireless transmitter module (600), wherein the wireless transmitter module is configured:
for user removable attachment to the flexible indwelling biosensor insertion mechanism; and
for receiving signals from the sensing element and transmitting the received signals in a wireless manner.

11. The flexible indwelling biosensor insertion device of claim 9 wherein the insertion mechanism includes a guide (508) configured for movement of the flexible indwelling biosensor therethrough during use of the flexible indwelling biosensor insertion device.

12. The flexible indwelling biosensor insertion device of claim 9 wherein the insertion mechanism is configured for curved insertion of a portion of the flexible indwelling biosensor, including at least the sharp head and the sensing element, into a target site.

## Patentansprüche

1. Flexibler Verweil-Biosensor (200), umfassend:
einen länglichen, aus einem flexiblen Material gebildeten Rahmen (202) mit
einem Grundkörper (203),
einem scharfen Kopf (210),
einem distalen Ende (204) und
einem proximalen Ende (206), sowie
einen in den länglichen Rahmen integrierten Biosensor (214), wobei
der längliche Rahmen ein länglicher Streifen mit einer vom distalen zum proximalen Ende verlaufenden Längsachse ist, und
wobei der längliche Streifen mindestens einen darin ausgebildeten Kanal (212) aufweist und der mindestens eine Kanal wenigstens teilweise parallel zu der Längsachse angeordnet ist,
wobei sich der Biosensor mindestens teilweise in dem mindestens einen Kanal befindet,
wobei der Biosensor ein Sensorelement (216) umfasst, welches über dem Grundkörper und/oder dem scharfen Kopf des länglichen Rahmens, angeordnet ist
wobei der scharfe Kopf am distalen Ende des länglichen Rahmens angeordnet ist und
wobei der scharfe Kopf und mindestens das Sensorelement des Biosensors zum Einbringen in eine Zielposition ausgebildet sind,
wobei der flexible Verweil-Biosensor weiterhin ein flexibles Rohr umfasst, welches den länglichen Rahmen und den Biosensor zwischen dem distalen und dem proximalen Ende des länglichen Rahmens mindestens teilweise ummantelt,
dadurch charakterisiert, dass das flexible Rohr einen C-förmigen Querschnitt aufweist, so dass das flexible Rohr den länglichen Rahmen teilweise umschließt.

2. Flexibler Verweil-Biosensor nach Anspruch 1, wobei das flexible Rohr so ausgebildet ist, dass zwischen dem flexiblen Rohr, dem Biosensor und dem länglichen Rahmen innerhalb des Kanals eine flüssigkeitsdichte Abdichtung besteht.

3. Flexibler Verweil-Biosensor nach Anspruch 1, wobei der Biosensor mindestens eine Signalübertragungsleitung, vorzugsweise zwei oder drei Signalübertragungsleitungen einschließt, welche jeweils
ein proximales Ende und
einen am proximalen Ende der Signalübertragungsleitung angeordneten Signalübertragungsstecker aufweisen.

4. Flexibler Verweil-Biosensor nach Anspruch 1, wobei der scharfe Kopf zur subkutanen Einführung ausgebildet ist und die Zielposition eine Zielposition in der Haut ist.

5. Flexibler Verweil-Biosensor nach Anspruch 1, wobei das flexible Material ein superelastisches flexibles Material, beispielsweise Nitinol, ist.

6. Flexibler Verweil-Biosensor nach Anspruch 1, wobei das Sensorelement auf dem scharfen Kopf und/oder dem Grundkörper des länglichen Rahmens oder darüber hängend angeordnet ist.

7. Flexibler Verweil-Biosensor nach Anspruch 1 oder 2, wobei das Sensorelement innerhalb des Kanals und auf dem Grundkörper des länglichen Rahmens oder darüber hängend angeordnet ist.

8. Flexibler Verweil-Biosensor nach Anspruch 1, wobei der Grundkörper des länglichen Rahmens eine Rahmenöffnung durch sich hindurch aufweist und das Sensorelement über der Rahmenöffnung angeordnet ist.

9. Einrichtung (500) zum Einführen des flexiblen Verweil-Biosensors, umfassend:
einen flexiblen Verweil-Biosensor (200) nach
einem der vorgenannten Ansprüche und
einen Einführungsmechanismus (502),
wirkverbunden mit dem flexiblen Verweil-Biosensor und in diesen integriert, wobei der Einführungsmechanismus zum Einführen eines Teils des flexiblen Verweil-Biosensors, einschließlich mindestens des scharfen Kopfes und des Sensorelements, in eine Zielposition ausgebildet ist.

10. Einrichtung zum Einführen des flexiblen Verweil-Biosensors nach Anspruch 9, weiterhin umfassend ein drahtloses Übertragungsmodul (600),
wobei das drahtlose Übertragungsmodul ausgebildet ist
zum Anbringen an den Einführungsmechanismus für den flexiblen Verweil-Biosensor, so dass das Modul vom Benutzer wieder entfernt werden kann, und
zum Empfangen von Signalen des Sensorelements und drahtlosen Senden der empfangenen Signale.

11. Einführungseinrichtung für den flexiblen Verweil-Biosensor nach Anspruch 9, wobei der Einführungsmechanismus eine Führung (508) enthält, welche dafür ausgebildet ist, dass der flexible Verweil-Biosensor bei Benutzung der Einführungseinrichtung für den flexiblen Verweil-Biosensor durch diese Führung bewegt wird.

12. Einführungseinrichtung für den flexiblen Verweil-Biosensor nach Anspruch 9, wobei der Einführungsmechanismus für eine kurvenförmige Einführung eines Teils des flexiblen Verweil-Biosensors, einschließlich mindestens des scharfen Kopfes und des Sensorelements, in eine Zielposition ausgebildet ist.

## Revendications

1. Biocapteur flexible à demeure (200) comprenant :
une structure allongée (202) formée à partir d'un matériau flexible, la structure allongée ayant :
une partie de corps (203) ;
une tête pointue (210) ;
une extrémité distale (204) ; et
une extrémité proximale (206) ; et
un biocapteur (214) intégré dans la structure allongée, dans lequel
la structure allongée est une bande allongée avec un axe longitudinal s'étendant depuis l'extrémité distale jusqu'à l'extrémité proximale ; et
dans lequel la bande allongée comporte au moins un canal (212) formé dans cette dernière, le ou les canaux étant disposés au moins partiellement parallèlement à l'axe longitudinal ; et
dans lequel le biocapteur est au moins partiellement contenu à l'intérieur du ou des canaux ;
le biocapteur comprenant :
un élément de détection (216) disposé sur la partie de corps et/ou la tête pointue de la structure allongée ;
dans lequel la tête pointue est disposée au niveau de l'extrémité distale de la structure allongée ; et
dans lequel la tête pointue et au moins l'élément de détection du biocapteur sont configurés pour une insertion dans un site cible ;
le biocapteur flexible à demeure comprenant en outre un tube flexible enrobant au moins partiellement la structure allongée et le biocapteur entre l'extrémité distale et l'extrémité proximale de la structure allongée,
**caractérisé en ce que** le tube flexible présente une section transversale en forme de C de telle sorte que le tube flexible entoure partiellement la structure allongée.

2. Biocapteur flexible à demeure selon la revendication 1, dans lequel le tube flexible est configuré pour fournir un joint étanche aux liquides entre le tube flexible, le biocapteur et la structure allongée dans le canal.

3. Biocapteur flexible à demeure selon la revendication 1, dans lequel le biocapteur comprend au moins une ligne de transmission de signaux, de préférence deux ou trois lignes de transmission de signaux, ayant chacune :
une extrémité proximale ; et
un connecteur de transmission de signaux disposé au niveau de l'extrémité proximale de la ligne de transmission de signaux.

4. Biocapteur flexible à demeure selon la revendication 1, dans lequel la tête pointue est configurée pour une insertion sous-cutanée et le site cible est un site cible de la peau.

5. Biocapteur flexible à demeure selon la revendication 1, dans lequel le matériau flexible est un matériau flexible super élastique, par exemple le Nitinol.

6. Biocapteur flexible à demeure selon la revendication 1, dans lequel l'élément de détection est disposé sur, ou pend au-dessus de, la tête pointue et/ou la partie de corps de la structure allongée.

7. Biocapteur flexible à demeure selon la revendication 1 ou la revendication 2, dans lequel l'élément de détection est disposé dans le canal et sur, ou suspendu au-dessus de, la partie de corps de la structure allongée.

8. Biocapteur flexible à demeure selon la revendication 1, dans lequel la partie de corps de la structure allongée comprend une ouverture de structure à travers cette dernière et l'élément de détection est disposé au-dessus de l'ouverture de structure.

9. Dispositif d'insertion de biocapteur flexible à demeure (500) comprenant :
un biocapteur flexible à demeure (200) selon l'une quelconque des revendications précédentes ; et
un mécanisme d'insertion (502) raccordé fonctionnellement au, et intégré dans le, biocapteur flexible à demeure, le mécanisme d'insertion étant configuré pour insérer une partie du biocapteur flexible à demeure, comprenant au moins la tête pointue et l'élément de détection, dans un site cible.

10. Dispositif d'insertion de biocapteur flexible à demeure selon la revendication 9, comprenant en outre un module émetteur sans fil (600),
dans lequel le module émetteur sans fil est configuré :
pour une fixation amovible par un utilisateur au mécanisme d'insertion de biocapteur flexible à demeure ; et
pour recevoir des signaux de l'élément de détection et transmettre les signaux reçus de manière sans fil.

11. Dispositif d'insertion de biocapteur flexible à demeure selon la revendication 9, dans lequel le mécanisme d'insertion comprend un guide (508) configuré pour permettre un mouvement du biocapteur flexible à demeure à travers ce dernier pendant l'utilisation du dispositif d'insertion de biocapteur flexible à demeure.

12. Dispositif d'insertion de biocapteur flexible à demeure selon la revendication 9, dans lequel le mécanisme d'insertion est configuré pour permettre une insertion en courbe d'une partie du biocapteur flexible à demeure, comprenant au moins la tête pointue et l'élément de détection, dans un site cible.
